# EUROPEAN PATENT SPECIFICATION

(11) **EP 1 897 563 B1**
(45) Date of publication and mention of the grant of the patent: **18.11.2009**
(21) Application number: 06120095.2
(22) Date of filing: 05.09.2006
(51) Int. Cl.: A61L 2/14, A61L 2/18, A61L 2/20, A43D 3/14

(54) **Shoe deodorizing apparatus**
Vorrichtung zum Desodorieren von Schuhen
Dispositif de désodorisation pour chaussures

(43) Date of publication of application: 12.03.2008
(73) Proprietor: Electrolux Home Products Corporation N.V., 1930 Zaventem (BE)
(72) Inventor: Fabbro, Edi, 33032 Bertiolo (Udine) (IT); Alexandrov, Sergei, 195251 St.-Petersburg (RU)
(74) Representative: Nardoni, Andrea

(56) References cited:
- FR-A1- 2 761 874
- US-A- 6 134 806
- US-A1- 2003 015 505
- US-B1- 6 585 935

## Description

The present invention refers to a shoe deodorizing apparatus.

Disclosed in US 6,565,805 is an ozone generator for deodorizing shoes, which comprises atmospheric-pressure discharge means adapted to convert air oxygen into ozone. The discharge means comprise a rough-surfaced dielectric element of rectangular shape, a first electrode, and a second electrode. The dielectric element is sandwiched between the first electrode and the second electrode, and both electrodes are connected to a high-frequency converter. The first electrode is comprised of a plurality of helical windings that contact a plurality of flanges on the dielectric element, and the second electrode is comprised of an electrically conductive coating which overlies the rough surface of the dielectric element. The rectangular shape of the dielectric element facilitates the alignment of the electrodes, and the flanges assist in maintaining this aligned position.

The oxygen contained in the air is converted into ozone by the action of corona discharges produced by the helical windings of the first electrode.

A major drawback encountered in the prior art is connected with the fact that ozone itself is not able to eliminate in an equally effective manner the different and widely varying types of organic substances and compounds that usually tend to infest shoes and footwear items in general and give rise to the well-known unpleasant smells. Chemically highly reactive molecular or atomic particles, such as radical groups, ions and atomic oxygen might in fact be used in view of enhancing the efficiency of prior-art shoe-deodorizing apparatuses, and might moreover be selected to decompose, disgregate and eliminate certain organic substances that fail to be removed by ozone.

Another drawback of prior-art apparatuses of the above-cited kind derives from the fact that ozone being generated to relatively high quantities and concentrations might ultimately prove as being potentially harmful to humans.

Further an high concentration of ozone tends to degrade plastics and rubbers which the shoes are made of.

The prior-art atmospheric-pressure discharge means are furthermore particularly complex from a construction point of view and scarcely effective from the viewpoint of their ability in dissociating and ionizing the gas/vapour molecules of the surrounding ambient.

US 2003/0015505 discloses an apparatus to sterilize objects by placing them between two electrodes using substantially atmospheric pressure plasma.

US 6 134 806 discloses a portable bag adapted to contain objects to be sanitized. The bag is inflated with sanitizing gas, which leaves the bag through proper openings.

FR 2 761 874 discloses a device using a disinfecting plate which causes the diffusion of a bactericide product. The plate only contains bactericides and deodorizing agents.

US 6585935 discloses a shoe deodorising apparatus comprising electrodes and a high-voltage supply for generating ions and active species.

It is therefore a main object of the present invention to provide a shoe deodorizing apparatus, which is effective in doing away with the above-noted drawbacks of the cited prior art.

Within this general object, it is another purpose of the present invention to provide a shoe deodorizing apparatus that, further to being simple in construction and convenient in use, is capable of ensuring an effective decomposition and elimination of a wide-ranging typology of organic substances and compounds as usually found in shoes.

According to the present invention, these aims, along with further ones that will become apparent from the following disclosure, are reached in a shoe deodorizing apparatus that incorporates the features as defined and recited in the claims appended hereto.

Features and advantages of the present invention will anyway be more readily understood from the description that is given below by way of nonlimiting example with reference to the accompanying drawings, in which:

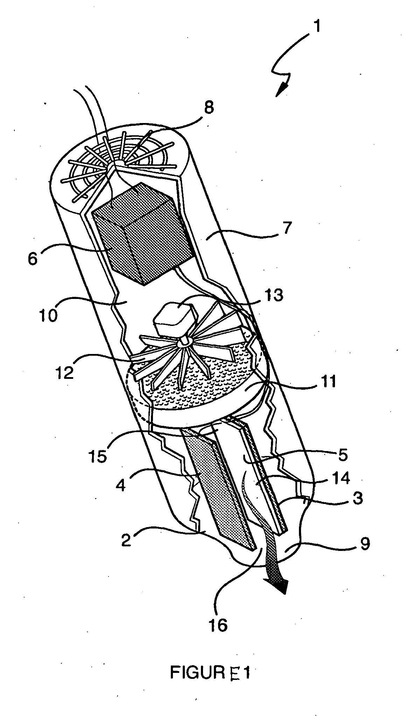

- Figure 1 is a perspective view of a shoe deodorizing apparatus according to the present invention, with the outer casing thereof shown in a cut-away view;
- Figure 2 is a perspective view of a shoe deodorizing apparatus according to another embodiment of the present invention, with the outer casing thereof shown again in a cut-away view;

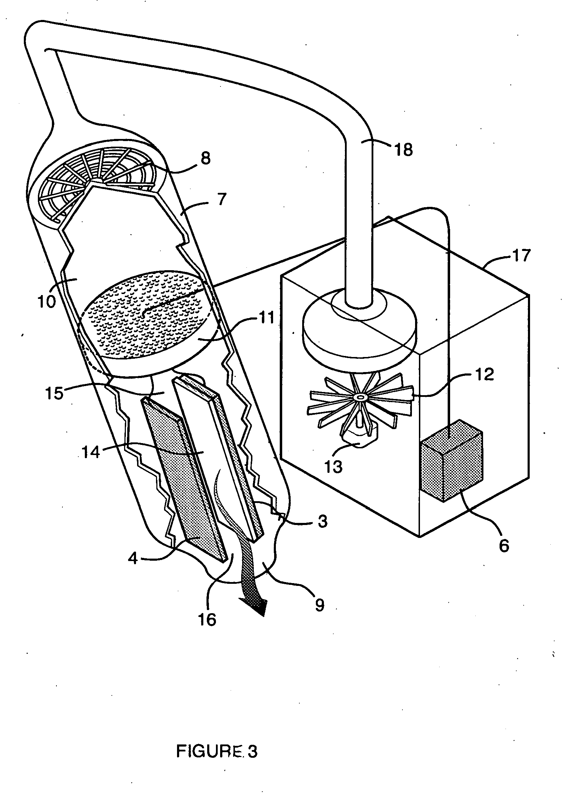

- Figure 3 is a perspective view of a shoe deodorizing apparatus according to a further embodiment of the present invention, with the outer casing thereof shown again in a cut-away view;
- Figure 4 is a perspective view of a shoe deodorizing apparatus according to a further embodiment yet of the present invention, with the outer casing thereof shown again in a cut-away view;
- Figure 5 is a perspective view of the atmospheric-pressure discharge assembly shown in Figures 2 and 4.
- Figure 6 is a perspective view of a shoe deodorizing apparatus according to a further embodiment of the present invention, with the outer casing thereof shown again in a cut-away view;

With reference to the above-cited Figures, the shoe deodorizing apparatus, as generally indicated with the reference numeral 1, comprises at least an atmospheric-pressure discharge assembly 2, 22 that includes a high-voltage electrode 3, 23, a counter-electrode 4, 24, and at least a dielectric element 5, 25 disposed therebetween, and it further comprises a high-voltage AC generator 6 connected to said high-voltage electrode 3, 23.

The apparatus further comprises a casing 7 provided with an intake port 8 and an exhaust port 9 so as to define a fluid passage 10 extending from the intake port 8 to the exhaust port 9, wherein said atmospheric-pressure discharge assembly 2,22, is arranged in the fluid passage 10.

The apparatus also comprises fluid supplying means for forcing an air stream along the fluid passage 10 and delivering means adapted to diffuse vapours or gas of predetermined substances into said air stream in such a way that the air stream is adapted to carry said vapours or gas to the atmospheric-pressure discharge assembly 2,22 where said vapours or gas are converted into chemically highly reactive particles adapted to decompose, disgregate organic compounds present in the shoe.

In a preferably embodiment of the present invention, the delivering means comprises porous means 11 soaked with liquid substances and arranged in the fluid passage 10 upstream of said atmospheric-pressure discharge assembly 2, 22, in such a way that the air stream is adapted to pass through the porous means 11 to take up vapours produced by the evaporation of said liquid substances and carry them with it to the atmospheric-pressure discharge assembly 2,22, where said vapours are converted into chemically highly reactive particles adapted to decompose, disgregate the organic compounds and substances that are present in the shoe.
In a most preferably embodiment the porous means 11 are provided in the fluid passage 10 inside said casing 7.

The casing 7 consists preferably of an elongated hollow body, which is advantageously made of an electrically insulating material, and which extends in a longitudinal direction and defines, at its end portions, the intake port 8 and the exhaust port 9, both of them fluidly connected with the ambient outside the casing 7, which is in turn so shaped and sized as to be able to be fully or at least partially inserted in a shoe to thereby allow the flow from the intake port 8 to be conveyed directly into the shoe.

The atmospheric-pressure discharge assembly 2, which is provided inside the outer casing near the exhaust port 9, is adapted to ionize, dissociate and excite the molecules of the vapours produced by the evaporation of the liquid substances impregnating the porous means 11, to thereby generate chemically highly reactive particles that destroy and eliminate the organic substances accumulated in the shoe, which give rise to the often unpleasant smell emanating from the shoes in the form of, say, volatile organic compounds, bacteria, moulds, fungi.

In the exemplary embodiment being considered to illustrative purposes, the fluid supplying means comprise a fan or impeller 12, which is arranged in the fluid passage 10 inside the outer casing 7 so as to be adapted to take in air through the intake port 8 to generate a stream of fluid flowing through and along the casing 7 and issuing therefrom through the exhaust port 9.

Such stream of fluid medium is adapted to pass through the porous means 11 and the atmospheric-pressure discharge assembly 2 before leaving the casing 7 through the exhaust port 9.

Preferably, the fan 12 is arranged inside the casing 7 in a position situated upstream from the porous means 11, so that it is therefore practically provided between the intake port 8 and the porous means themselves. However, it can be readily appreciated that the fan 12 may be provided inside the casing in any other position between the intake port 8 and the exhaust port 9, as well.

The motor means 13 used to drive the fan may themselves be provided inside the outer casing 7 in the fluid passage 10.

The motor means 13 may be powered directly from the power supply line or, preferably, they may be energized by means of a power supply to be possibly provided inside the casing 8, as well.

Such built-in power supply would preferably comprise a storage battery, such as in particular a rechargeable storage battery, so as to supply the fan 12 in an autonomous manner.

The porous means 11 may advantageously be comprised of porous absorbent materials, such as sponges or the like, that are capable of retaining liquid substances, while allowing air to pass therethrough. These materials are impregnated with appropriately selected liquid substances, which tend to naturally evaporate at room temperature, and whose evaporation is favoured by the air stream being forced to flow through the porous means. The molecules of the resulting vapour are carried by such airflow streaming therethrough up to the atmospheric-pressure discharge assembly 2, where they are ionized, dissociated and excited, to thereby generate chemically very reactive particles that are adapted to decompose and inactivate the organic substances and compounds originating the unpleasant smell of the shoes.

It has been experimentally found that, among various media investigated, the liquid substances that most of all seem to suit this application include water, which delivers OH-, H+; alcohols (R-OH), which deliver OH⁻ groups; formic acid (HCOOH) or formaldehyde (HCOH), which deliver OH⁻, H⁺, O atomic; freons, which deliver Cl⁻, F⁻, Br⁻; ammonia (NH₃) and hydrazine (N₂H₄), which deliver amino groups. Anyhow, the liquid substances to be used may of course be selected depending on the particular kind of organic compounds to be eliminated.

Advantageously, the porous means 11 can be periodically replaced through a proper access aperture provided in the casing 7, so as to maintain their capability of retaining liquid substances and allowing the passage of air substantially unaltered in time.

In addition, through-bores may be provided in the casing 7 allowing access to the porous means 11 for liquid substances to be added thereinto in view of restoring the same porous means 11 to the desired impregnation level, wherein the liquid substances may be added with the use of appropriate tools, such as a syringe. Opening/closing means are of course provided to open and close in a fluid-tight manner such bores, so as to effectively prevent air from leaking out of the casing 7 when the fan 12 is operated to force an air stream along the fluid passage 10.

As an alternative thereto, a drawer-like pull-out support may for instance be provided, which is adapted to both hold the porous means in place inside the casing - obviously without being of any hindrance to the free passage of the air stream through the porous means 11 - and to be pulled out of the casing whenever this is required in view of enabling the porous means 11 themselves to be replaced or the impregnation thereof to be restored through the addition of properly selected liquid substances.

Again, the porous means 11 may alternatively be provided in the form of replaceable cartridges - duly pre-soaked with the desired liquid substances - to be inserted in and removed from the casing 7 through an appropriate aperture.

In a particularly preferred embodiment of the present invention, the high-voltage electrode 3 and the counter-electrode 4 are constituted by a pair of mutually facing and spaced parallel-planar or plate electrodes, whereas the dielectric element 5 comprises a coating layer provided to cover the surface of the high-voltage electrode 3 facing the counter-electrode 4, so as to create a discharge gap 14 between the electrodes 3,4, where the chemically highly reactive particles are generated. In practice, such discharge gap 14 defines a fluid channel comprising an inlet section 15, adapted to let in the flow of air saturated with the vapour it took up from the liquid substances in the porous means 11, and an outlet section 16 provided in proximity to the exhaust port 9, where the chemically highly reactive particles generated along the path through the discharge gap 14 are eventually issuing from.

When an AC voltage is applied to the high-voltage electrode 3, a dielectric-barrier discharge (DBD) appears in the discharge gap 14 between the two electrodes 3,4.

Such DBD consists of a number of self-pulsing micro-discharges in the fluid between the electrodes, with corresponding surface discharges on the dielectric. The time constant of these processes are in the nanosecond region. The electrical energy coupled into the gas is mainly used to produce energetic electrons, while the gas remains at nearly ambient temperature.

The molecules of the vapours of the liquid substances that flow between the parallel-plate electrodes, owing to them colliding with the energetic electrons, are ionized, dissociated and excited, thereby generating active species, i.e. the afore-mentioned chemically highly reactive particles.

The high-voltage AC generator 6, which is connected to the high-voltage electrode 3, may be housed within the casing 7.

In an advantageous manner, the counter-electrode 4 is connected to ground.

The high-voltage AC generator 6 may be powered in an autonomous manner through the rechargeable storage battery provided inside the casing 7. However, the high-voltage AC generator 6 may be supplied directly from the power supply line or a low-voltage source located outside the same casing 7.

Switching means are provided to energize said high-voltage AC generator 6 and operate said fluid supplying means.
These switching means may include, for instance, a push-button, which is actuatably provided on the outer casing 7 to control circuit means connected to the power supply source in view of energizing/de-energizing the high-voltage electrode 3 and switching on/off the fan 12 as desired by the user.

Advantageously, the switching means are such as to enable the high-voltage electrode 3 to be energized and the fan 12 to be switched on at the same time and, conversely, the high-voltage electrode 3 to be de-energized and the fan 12 to be switched off at the same time.

In an alternative embodiment of the present invention, the high-voltage AC generator 6 is provided externally to the casing 7 so as to be capable of being connected to the power supply line directly.

In a further embodiment of the present invention, also the fluid supplying means are provided externally to the casing 7.

In particular, the high-voltage AC generator 6 and the fluid supplying means are housed in an appropriate station 17 provided separately from the casing 7.

The high-voltage AC generator 6 is connected to the high-voltage electrode 3 via suitable electric cables, whereas the fluid supplying means are fluidly connected to the intake port 8 and the fluid passage 10 via a fluid-carrying conduit 18. In practice, the fan 12 housed in the separate station 17 is adapted to generate an air stream that flows through the fluid-carrying conduit 18 and, passing through the intake port 8, moves along the fluid passage 10 to eventually take up the vapours of the liquid substances impregnating the porous media when passing therethrough, and carry such vapours with it through the discharge gap 14.

It is fully apparent that as an alternative, the porous means 11 can be arranged inside the station 17 in such a way that the air stream, forced by the fan 12 along the fluid-carrying conduit 18, is adapted to pass through the porous means 11 so as to the carry the vapours to the atmospheric-pressure discharge assembly 2. The porous means 11 can be disposed, as an example, in correspondence to an expanded inlet section of the fluid-carrying conduit 18 downstream the fan 12.

Means for allowing the impregnation and the replacement of the porous means 11 can be provided as described above for the embodiment in which the porous means are arranged inside the casing 7.

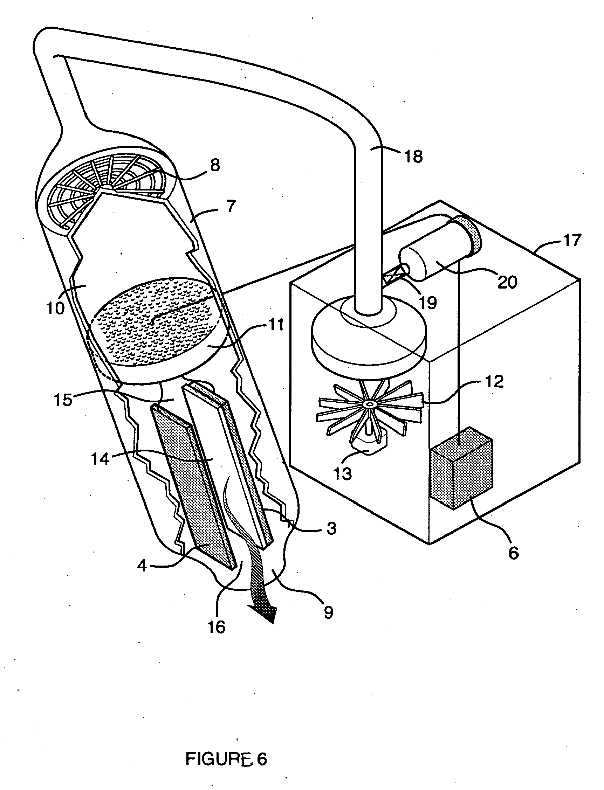

In a further embodiment of the present invention, shown in figure 6, the delivering means can comprise a reservoir 20 containing said vapours or gas compressed and adapted to issue said vapours or gas in the air stream. In particular, the reservoir 20 is housed in the station 17 and is fluidly connected to the fluid-carrying conduit 18 through a diffuser 19, which, for example, can be actuated by valve means. In an advantageous manner the switching means that enables the high-voltage electrode 3 to be energized are adapted to operate the valve means.

It can be readily appreciated that the reservoir can be arranged directly in the fluid passage 10 inside the casing 7.

In a further embodiment of the present invention, the delivering means can comprise a vaporizer or nebulizer fluidly connected to a reservoir, which contains predetermined liquid substance, for producing and diffusing said vapours or gas into the air stream. Preferably the vaporizer or nebulizer is housed inside the station 17 and fluidly connected to the fluid-carrying conduit 18. Clearly, the vaporizer or nebulizer can be arranged directly in the fluid passage 10 inside the casing 7.

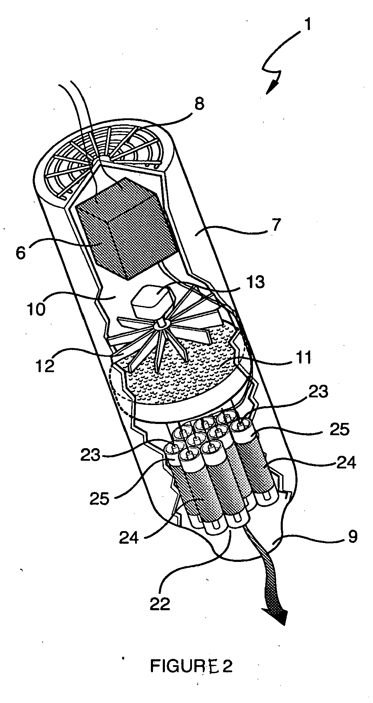

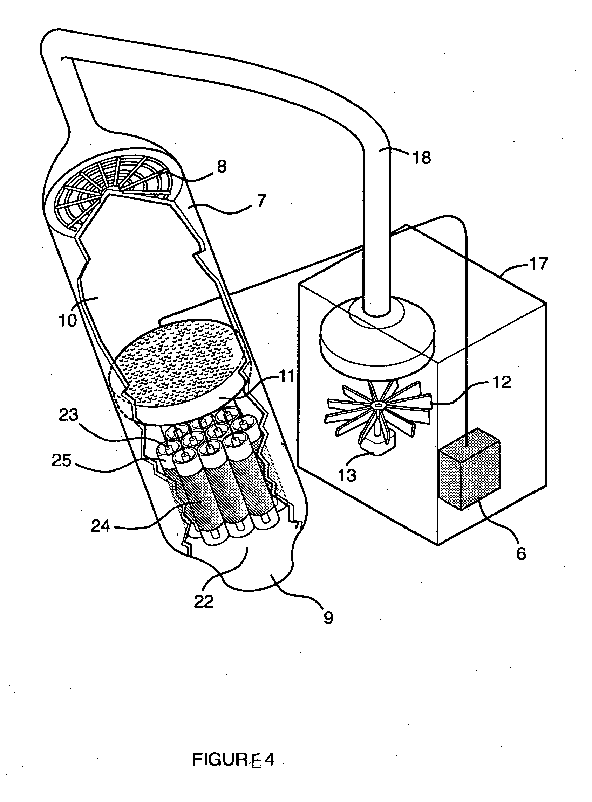

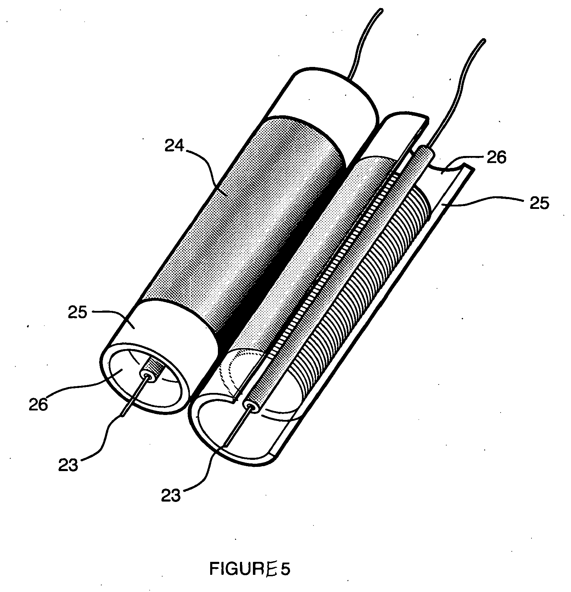

In a yet further embodiment of the present invention, the dielectric-barrier discharge assembly 22, as best shown in Figures 2, 4, 5, comprises a dielectric element 25 consisting of a hollow cylinder of a dieletric material, such as preferably glass, inside which there is coaxially arranged a high-voltage electrode 23, which is provided in the form of a rod-like body connected to the high-voltage AC generator 6, and extends along the axis of the cylinder of the dielectric element 25, and a counter-electrode 24, which is provided in the form of a metal foil, is connected preferably to ground, and is wound round the outer surface of the cylinder of the dielectric element 25.

The annular space comprised between the dielectric element 25 and the high-voltage electrode 23 forms the discharge gap 26, through which there moves the flow of vapours from the liquid substances in the porous media to be converted into chemically highly reactive particles.

In an advantageous manner, a plurality of dielectric-barrier discharge assemblies 22 are provided within the casing 7, where they are fitted together juxtaposed to each other and with the respective counter-electrodes 24 in mutual contact.

Also with this different configuration of the dielectric-barrier discharge assembly 2 it is contemplated that the high-voltage AC generator 6 and the fluid supplying means be provided outside the casing 7, in a separate station 17, in the same manner as already described hereinbefore in connection with the embodiment based on the use of a pair of parallel-planar electrodes.

It has been experimentally found that a supply voltage in the order of 2.5 to 3.0 kV for the high-voltage electrode, 3, 23 is adequate in view of ensuring a minimum formation of ozone in the flow of air saturated with the vapours, thereby preventing dangerous ozone concentrations from forming to the detriment of humans.

Conclusively, it can therefore be stated that the shoe deodorizing apparatus according to the present invention ensures an effective removal of the most varied organic substances and compounds that may be present in shoes, and is able to do this in an extremely simple and convenient manner, thereby doing away with the serious drawback shared by prior- art devices.

The apparatus according to the present invention is such as to ensure a steady, sizeable flow of vapours of the liquid substances in the porous media through the discharge gap, thereby favouring the desired active species, i.e. the chemically highly reactive particles to be generated at a particularly high rate. For such active species to be generated at an adequately high rate, it is fact quite important to have a substantial stream of aeriform flowing through the discharge gap, actually.

Although featuring an extremely simple construction, the atmospheric-pressure discharge assembly of the present invention is capable of delivering a very high dissociation and ionization energy to the vapour molecules traveling along the discharge gap, thereby generating chemically highly reactive particles at a particularly high rate.

Furthermore, since the dissociation and ionization processes and, more generally, the energy transfer processes from the micro-discharges to the fluid prevailingly involve the vapours of the liquid substances being carried in the same fluid, only a limited amount of energy remains available to convert oxygen from the air into ozone. Ozone is therefore produced to a quite limited, generally very low amount.

## Claims

1. Shoe deodorizing apparatus comprising at least
- an atmospheric-pressure discharge assembly (2, 22) including a high-voltage electrode (3, 23), a counter-electrode (4, 24), and at least a dielectric element (5, 25) disposed therebetween,
- a high-voltage AC generator (6) connected to said high-voltage electrode (3,23),
- a casing (7) provided with an intake port (8) and an exhaust port (9) so as to define a fluid passage (10) extending from the intake port (8) to the exhaust port (9), wherein said atmospheric-pressure discharge assembly (2, 22) is arranged in the fluid passage (10),
- fluid supplying means for forcing an air stream along the fluid passage (10),
said casing (7) being so shaped and sized as to be able to be fully or at least partially inserted in a shoe to thereby allow the flow of said chemically highly reactive particles to be conveyed directly into the shoe through the exhaust port (9) **characterized by** further comprising delivering means adapted to diffuser vapours or gas of predetermined substances into said air stream in such a way that the air stream is adapted to carry said vapours or gas to the atmospheric-pressure discharge assembly (2, 22) for generating, out of said vapours or gas, chemically highly reactive particles adapted to decompose organic compounds present in the shoe.

2. Apparatus according to claim 1, wherein said delivering means comprises porous means (11) impregnated with predetermined liquid substances and arranged upstream of said atmospheric-pressure discharge assembly (2,22) in such a way that the air stream is adapted to pass through the porous means (11) to carry vapours produced by the evaporation of said liquid substances to the atmospheric-pressure discharge assembly (2,22) for generating, out of said vapours, chemically highly reactive particles adapted to decompose organic compounds present in the shoe.

3. Apparatus according to claim 1, wherein said delivering means comprise a reservoir (20) containing said vapours or gas compressed and adapted to issue said.vapours or gas into the air stream.

4. Apparatus according to claim 1, wherein said delivering means comprise a vaporizer or nebulizer fluidly connected to a reservoir containing predetermined liquid substances to produce said vapours or gas.

5. Apparatus according to claim 2, wherein said porous means (11) are provided in the fluid passage (10) inside said casing (7).

6. Apparatus according to claim 5, wherein said casing (7) is provided with at least an access aperture allowing access to be gained to said porous means (11), so as to be able to impregnate said porous means (11) with said liquid substances and/or to replace said porous means (11).

7. Apparatus according to claim 5, wherein said casing (7) is provided with through-bores allowing access to be gained to the porous means (11) so as to enable the liquid substances to be added from the outside and the porous means (11) to be impregnated with such liquid substances.

8. Apparatus according to claim 5, wherein a pull-out support is provided to hold the porous means (11) in place within the casing (7), said support being adapted to be pulled out of the casing (7) to allow the porous means (11) to be replaced and/or the porous means (11) to be impregnated with the liquid substances.

9. Apparatus according to claim 5, wherein said porous means (11) are provided in the form of replaceable cartridges, duly pre-soaked with the desired liquid substances, to be introduced into and removed from the casing (7) through an appropriate aperture.

10. Apparatus according to any of the preceding claims, wherein said fluid supplying means comprise a fan or impeller (12).

11. Apparatus according claims 10, wherein said fan is provided in the fluid passage (10) inside the outer casing (7).

12. Apparatus according to any of the preceding claims 1-9, wherein said fluid supplying means are provided outside the casing (7) and are fluidly connected to the intake port (8) and the fluid passage (10) via a fluid-carrying conduit (18).

13. Apparatus according to any of the preceding claims, wherein switching means are provided to energize said high-voltage AC generator (6) and operate said fluid supplying means.

14. Apparatus according to any of the preceding claims, wherein the high-voltage electrode (3) and the counter-electrode (4) are comprised of a pair of mutually facing and spaced parallel-planar or plate electrodes, whereas the dielectric element (5) is comprised of a coating layer covering the surface of the high-voltage electrode (3) facing the counter-electrode (4) so as to form a discharge gap (14) between the electrodes (3,4), where the chemically highly reactive particles are generated.

15. Apparatus according to claim 14, wherein said discharge gap (14) defines a fluid-carrying channel comprising an inlet section (15) adapted to receive the air stream saturated with the vapour from the liquid substances, and an outlet section (16) provided in proximity to the exhaust port (9,) where the chemically highly reactive particles generated along the discharge gap (14) are let out from.

16. Apparatus according to any of the preceding claims 1 to 13, wherein said atmospheric-pressure discharge assembly (22) comprises a dielectric element (25) consisting of a hollow cylinder, a high-voltage electrode (23) consisting of a rod-like body provided inside the cylinder along the axis thereof, and a counter-electrode (24) consisting of a metal foil wound round the outer surface of the cylinder of the dielectric barrier (25).

17. Apparatus according to claim 16, wherein a plurality of atmospheric-pressure discharge assemblies (22) are provided inside the casing (7) in a juxtaposed arrangement relative to each other with the respective counter-electrodes (24) in contact with each other.

18. Apparatus according to any of the preceding claims, wherein said high-voltage electrode (3, 23) is supplied with a power-supply voltage having a value situated between 2.5 and 3.0 kv to ensure a minimum ozone formation in the plasma.

19. Method for deodorizing shoes comprising:
- providing at least an atmospheric-pressure discharge assembly (2,22) including a high-voltage electrode (3,23), a counter-electrode (4,24), and at least a dielectric element (5,25) disposed therebetween, and further comprising a high-voltage AC generator (6) connected to said high-voltage electrode (3,23), whereby it comprises the step of:
- providing a casing (7) with an intake port (8) and an exhaust port (9) so as to define a fluid passage (10) extending from the intake port (8) to the exhaust port (9), wherein said atmospheric-pressure discharge assembly (2,22) is arranged in the fluid passage (10), said casing (7) being so shaped and sized as to be able to be fully or at least partially inserted in a shoe;
- placing the casing in a shoe,
- energizing the high-voltage AC generator (6),
- forcing an air stream along the fluid passage (10), and diffusing vapours or gas of predetermined substances into said air stream in such a way that the air stream is adapted to carry said vapours or gas to the atmospheric-pressure discharge assembly (2,22) for generating, out of said vapours or gas, chemically highly reactive particles,
thereby allowing the flow of said chemically highly reactive particles to be conveyed directly into the shoe through the exhaust port (9) to for decomposing organic compounds present in the shoe.

## Patentansprüche

1. Vorrichtung zum Desodorieren von Schuhen, mindestens umfassend:
- eine Atmosphärendruck-Ausstoßanordnung (2, 22) mit einer Hochspannungselektrode (3, 23), einer Gegenelektrode (4, 24) und mindestens einem dazwischen angeordneten dielektrischen Element (5, 25),
- einen Hochspannungs-Wechselstromgenerator (6), der mit der Hochspannungselektrode (3, 23) verbunden ist,
- ein Gehäuse (7), das mit einer Einlassöffnung (8) und einer Auslassöffnung (9) versehen ist, um einen Fluidkanal (10) zu definieren, der sich von der Einlassöffnung (8) zur Auslassöffnung (9) erstreckt, wobei die Atmosphärendruck-Ausstoßanordnung (2, 22) in dem Fluidkanal (10) angeordnet ist,
- Fluidzufuhrmittel, um einen Luftstrom den Fluidkanal (10) entlangzublasen,
wobei das Gehäuse (7) derart geformt und bemessen ist, dass es geeignet ist, vollständig oder zumindest teilweise in einen Schuh eingeführt zu werden, um es **dadurch** einem Strom chemisch hochreaktiven Partikel zu erlauben, durch die Auslassöffnung (9) direkt in den Schuh befördert zu werden, **dadurch gekennzeichnet, dass** sie darüber hinaus Abgabemittel umfasst, die geeignet sind, Dämpfe oder Gase von vorbestimmten Substanzen in dem Luftstrom zu verteilen, so dass der Luftstrom die Dämpfe oder Gase zur Atmosphärendruck-Ausstoßanordnung (2, 22) befördern kann, um aus den Dämpfen oder Gasen chemisch hochreaktive Partikel zu erzeugen, die geeignet sind, in dem Schuh gegenwärtige organische Verbindungen zu zersetzen.

2. Vorrichtung nach Anspruch 1, wobei die Abgabemittel poröse Mittel (11) umfassen, die mit vorbestimmten flüssigen Substanzen imprägniert sind und stromaufwärts von der Atmosphärendruck-Ausstoßanordnung (2, 22) angeordnet sind, so dass der Luftstrom durch die porösen Mittel (11) dringen kann, um die durch die Verdunstung der flüssigen Substanzen entstandenen Dämpfe zur Atmosphärendruck-Ausstoßanordnung (2, 22) zu befördern, um aus den Dämpfen oder Gasen chemisch hochreaktive Partikel zu erzeugen, die geeignet sind, in dem Schuh gegenwärtige organische Verbindungen zu zersetzen.

3. Vorrichtung nach Anspruch 1, wobei die Abgabemittel einen Behälter (20) umfassen, der die verdichteten Dämpfe oder Gase enthält und der geeignet ist, die Dämpfe oder Gase in den Luftstrom abzugeben.

4. Vorrichtung nach Anspruch 1, wobei die Abgabemittel einen Zerstäuber oder Vernebler umfassen, der in Fluidverbindung mit einem Behälter steht, der die vorbestimmten flüssigen Substanzen enthält, um die Dämpfe oder Gase zu erzeugen.

5. Vorrichtung nach Anspruch 2, wobei die porösen Mittel (11) im Fluidkanal (10) im Inneren des Gehäuses (7) vorgesehen sind.

6. Vorrichtung nach Anspruch 5, wobei das Gehäuse (7) mit mindestens einer Zugangsöffnung versehen ist, die Zugang zu den porösen Mitteln (11) verschafft, um die porösen Mittel (11) mit den flüssigen Substanzen imprägnieren zu können und/oder die porösen Mittel (11) austauschen zu können.

7. Vorrichtung nach Anspruch 5, wobei das Gehäuse (7) mit Durchgangsbohrungen versehen ist, die Zugang zu den porösen Mitteln (11) verschaffen, um von außen die flüssigen Substanzen zugeben zu können und die porösen Mittel (11) mit solchen flüssigen Substanzen imprägnieren zu können.

8. Vorrichtung nach Anspruch 5, wobei ein ausziehbarer Träger vorgesehen ist, um die porösen Mittel (11) an ihrem Platz im Gehäuse (7) zu halten, wobei der Träger geeignet ist, aus dem Gehäuse (7) gezogen zu werden, um die porösen Mittel (11) austauschen zu können und/oder die porösen Mittel (11) mit den flüssigen Substanzen imprägnieren zu können.

9. Vorrichtung nach Anspruch 5, wobei die porösen Mittel (11) in Form austauschbarer Patronen vorgesehen sind, die im Voraus entsprechend mit den gewünschten flüssigen Substanzen getränkt wurden und die durch eine geeignete Öffnung in das Gehäuse (7) einzuführen und aus diesem zu entfernen sind.

10. Vorrichtung nach einem der vorhergehenden Ansprüche, wobei die Fluidzufuhrmittel einen Ventilator oder ein Gebläserad (12) umfassen.

11. Verfahren nach Anspruch 10, wobei der Ventilator in dem Fluidkanal (10) innerhalb des Außengehäuses (7) vorgesehen ist.

12. Verfahren nach einem der Ansprüche 1 - 9, wobei die Fluidzufuhrmittel außerhalb des Gehäuses (7) vorgesehen sind und über eine Fluidtransportleitung (18) in Fluidverbindung mit der Einlassöffnung (8) und dem Fluidkanal (10) stehen.

13. Vorrichtung nach einem der vorhergehenden Ansprüche, wobei Schaltmittel vorgesehen sind, um den Hochspannungs-Wechselstromgenerator (6) einzuschalten und die Fluidzufuhrmittel zu betreiben.

14. Vorrichtung nach einem der vorhergehenden Ansprüche, wobei die Hochspannungselektrode (3) und die Gegenelektrode (4) aus einem Paar einander gegenüberliegender und voneinander beabstandeter planparalleler Elektroden oder Plattenelektroden gebildet sind, während das dielektrische Element (5) aus einer Überzugsschicht gebildet ist, die die Fläche der Hochspannungselektrode (3) bedeckt, die der Gegenelektrode (4) gegenüberliegt, um eine Entladestrecke (14) zwischen den Elektroden (3, 4) zu bilden, wo die chemisch hochreaktiven Partikel erzeugt werden.

15. Vorrichtung nach Anspruch 14, wobei die Entladestrecke (14) einen Fluidbeförderungskanal bildet, der Folgendes umfasst: einen Einlassabschnitt (15), der geeignet ist, den Luftstrom aufzunehmen, der mit dem Dampf aus den flüssigen Substanzen gesättigt ist, und einen Auslassabschnitt (16), der in der Nähe der Auslassöffnung (9) vorgesehen ist, wo die chemisch hochreaktiven Partikel, die entlang der Entladestrecke (14) gebildet werden, ausgestoßen werden.

16. Vorrichtung nach einem der vorhergehenden Ansprüche 1 bis 13, wobei die Atmosphärendruck-Ausstoßanordnung (22) Folgendes umfasst: ein dielektrisches Element (25), das aus einem hohlen Zylinder gebildet ist, eine Hochspannungselektrode (23), die aus einem stabförmigen Körper gebildet ist, der in dem Zylinder entlang von dessen Achse vorgesehen ist, und eine Gegenelektrode (24), die aus einer Metallfolie gebildet ist, die um die Außenfläche des Zylinders der dielektrischen Barriere (25) gewickelt ist.

17. Vorrichtung nach Anspruch 16, wobei eine Vielzahl von Atmosphärendruck-Ausstoßanordnungen (22) in dem Gehäuse (7) nebeneinander angeordnet sind, wobei die jeweiligen Gegenelektroden (24) in Kontakt miteinander sind.

18. Vorrichtung nach einem der vorhergehenden Ansprüche, wobei die Hochspannungselektrode (3, 23) mit einer Versorgungsspannung versorgt wird, deren Wert zwischen 2,5 und 3,0 kV liegt, um eine minimale Ozonbildung im Plasma zu gewährleisten.

19. Verfahren zum Desodorieren von Schuhen, umfassend:
- Bereitstellen von mindestens einer Atmosphärendruck-Ausstoßanordnung (2, 22) mit einer Hochspannungselektrode (3, 23), einer Gegenelektrode (4, 24) und mindestens einem dazwischen angeordneten dielektrischen Element (5, 25), und darüber hinaus umfassend einen Hochspannungs-Wechselstromgenerator (6), der mit der Hochspannungselektrode (3, 23) verbunden ist, wobei es folgende Schritte umfasst:
- Bereitstellen eines Gehäuses (7) mit einer Einlassöffnung (8) und einer Auslassöffnung (9), um einen Fluidkanal (10) zu definieren, der sich von der Einlassöffnung (8) zur Auslassöffnung (9) erstreckt, wobei die Atmosphärendruck-Ausstoßanordnung (2, 22) in dem Fluidkanal (10) angeordnet ist,
wobei das Gehäuse (7) derart geformt und bemessen ist, dass es geeignet ist, vollständig oder zumindest teilweise in einen Schuh eingeführt zu werden,
- Anordnen des Gehäuses in einem Schuh,
- Einschalten des Hochspannungs-Wechselstromgenerators (6),
- Blasen eines Luftstroms entlang dem Fluidkanal (10) und Verteilen von Dämpfen oder Gasen vorbestimmter Substanzen im Luftstrom, so dass der Luftstrom die Dämpfe oder Gase zur Atmosphärendruck-Ausstoßanordnung (2,22) befördern kann, um aus den Dämpfen oder Gasen chemisch hochreaktive Partikel zu erzeugen,
wodurch es möglich wird, den Strom chemisch hochreaktiver Partikel durch die Auslassöffnung (9) direkt in den Schuh zu befördern, um in dem Schuh gegenwärtige organische Verbindungen zu zersetzen.

## Revendications

1. Appareil à désodoriser les chaussures comprenant au moins
- un ensemble de décharge à pression atmosphérique (2, 22) comprenant une électrode haute tension (3, 23), une contre-électrode (4, 24), et au moins un élément diélectrique (5, 25) disposé entre elles,
- un générateur CA haute tension (6) connecté à ladite électrode haute tension (3, 23),
- un boîtier (7) doté d'un orifice d'admission (8) et d'un orifice d'échappement (9) de manière à définir un passage de fluide (10) s'étendant de l'orifice d'admission (8) vers l'orifice d'échappement (9), dans lequel ledit ensemble de décharge à pression atmosphérique (2, 22) est agencé dans le passage de fluide (10),
- un moyen d'alimentation en fluide pour forcer un courant d'air le long du passage de fluide (10), ledit boîtier (7) étant formé et dimensionné de manière à pouvoir être inséré complètement ou au moins partiellement dans une chaussure pour permettre ainsi l'écoulement desdites particules chimiquement hautement réactives à transporter directement dans la chaussure par l'intermédiaire de l'orifice d'échappement (9), **caractérisé par le fait qu'**il comprend en outre
- un moyen de distribution adapté pour diffuser des vapeurs ou du gaz de substances prédéterminées dans ledit courant d'air de telle manière que le courant d'air est adapté pour transporter lesdites vapeurs ou ledit gaz vers l'ensemble de décharge à pression atmosphérique (2, 22) pour générer, à partir desdites vapeurs ou dudit gaz, des particules chimiquement hautement réactives adaptées pour décomposer des composés organiques présents dans la chaussure.

2. Appareil selon la revendication 1, dans lequel ledit moyen de distribution comprend un moyen poreux (11) imprégné de substances liquides prédéterminées et agencé en amont dudit ensemble de décharge à pression atmosphérique (2, 22) de telle manière que le courant d'air est adapté pour traverser le moyen poreux (11) pour transporter des vapeurs produites par l'évaporation desdites substances liquides vers l'ensemble de décharge à pression atmosphérique (2, 22), pour générer, à partir desdites vapeurs, des particules chimiquement hautement réactives adaptées pour décomposer des composés organiques présents dans la chaussure.

3. Appareil selon la revendication 1, dans lequel ledit moyen de distribution comprend un réservoir (20) contenant lesdites vapeurs ou ledit gaz comprimé et adapté pour faire sortir lesdites vapeurs ou ledit gaz dans le courant d'air.

4. Appareil selon la revendication 1, dans lequel ledit moyen de distribution comprend un vaporiseur ou un pulvérisateur raccordé de manière fluidique à un réservoir contenant des substances liquides prédéterminées pour produire lesdites vapeurs ou ledit gaz.

5. Appareil selon la revendication 2, dans lequel ledit moyen poreux (11) est disposé dans le passage de fluide (10) à l'intérieur dudit boîtier (7).

6. Appareil selon la revendication 5, dans lequel ledit boîtier (7) est doté d'au moins une ouverture d'accès permettant d'accéder audit moyen poreux (11) de manière à pouvoir imprégner ledit moyen poreux (11) avec lesdites substances liquides et/ou remplacer ledit moyen poreux (11).

7. Appareil selon la revendication 5, dans lequel ledit boîtier (7) est doté d'alésages traversants permettant d'accéder au moyen poreux (11) de manière à permettre aux substances liquides d'être ajoutées à partir de l'extérieur et au moyen poreux (11) d'être imprégné avec de telles substances liquides.

8. Appareil selon la revendication 5, dans lequel un support extractible est prévu pour maintenir le moyen poreux (11) en place dans le boîtier (7), ledit support étant adapté pour être extrait du boîtier (7) pour permettre au moyen poreux (11) d'être remplacé et/ou au moyen poreux (11) d'être imprégné avec les substances liquides.

9. Appareil selon la revendication 5, dans lequel ledit moyen poreux (11) est prévu sous la forme de cartouches remplaçables et pré-trempées comme il convient avec les substances liquides souhaitées, pour être introduit dans le boîtier (7) ou retiré de celui-ci à travers une ouverture appropriée.

10. Appareil selon l'une quelconque des revendications précédentes, dans lequel ledit moyen d'alimentation en fluide comprend une soufflante ou une turbine (12).

11. Appareil selon la revendication 10, dans lequel ladite soufflante est disposée dans le passage de fluide (10) à l'intérieur du boîtier externe (7).

12. Appareil selon l'une quelconque des revendications précédentes 1 à 9, dans lequel ledit moyen d'alimentation en fluide est disposé à l'extérieur du boîtier (7) et est raccordé de manière fluidique à l'orifice d'admission (8) et au passage de fluide (10) via un conduit acheminant le fluide (18).

13. Appareil selon l'une quelconque des revendications précédentes, dans lequel des moyens de commande sont prévus pour alimenter ledit générateur CA haute tension (6) et actionner ledit moyen d'alimentation en fluide.

14. Appareil selon l'une quelconque des revendications précédentes, dans lequel l'électrode haute tension (3) et la contre-électrode (4) sont composées d'une paire d'électrodes en plaque ou parallèles planes espacées et se faisant mutuellement face, tandis que l'élément diélectrique (5) est composé d'une couche de revêtement recouvrant la surface de l'électrode haute tension (3) faisant face à la contre-électrode (4) de manière à former un entrefer de décharge (14) entre les électrodes (3, 4), où les particules chimiquement hautement réactives sont générées.

15. Appareil selon la revendication 14, dans lequel ledit entrefer de décharge (14) définit un canal acheminant le fluide comprenant une section d'entrée (15) adaptée pour recevoir le courant d'air saturé avec la vapeur provenant des substances liquides, et une section de sortie (16) disposée à proximité de l'orifice d'échappement (9), d'où sortent les particules chimiquement hautement réactives générées le long de l'entrefer de décharge (14).

16. Appareil selon l'une quelconque des revendications précédentes 1 à 13, dans lequel ledit ensemble de décharge à pression atmosphérique (22) comprend un élément diélectrique (25) constitué d'un cylindre creux, d'une électrode haute tension (23) consistant en un corps de type tige disposé à l'intérieur du cylindre le long de son axe, et d'une contre-électrode (24) constituée d'une feuille de métal enroulée autour de la surface externe du cylindre de la barrière diélectrique (25).

17. Appareil selon la revendication 16, dans lequel une pluralité d'ensembles de décharge à pression atmosphérique (22) est disposée à l'intérieur du boîtier (7) dans un agencement juxtaposé les uns par rapport aux autres, les contre-électrodes (24) respectives étant en contact les unes avec les autres.

18. Appareil selon l'une quelconque des revendications précédentes, dans lequel ladite électrode haute tension (3, 23) est alimentée en tension d'alimentation ayant une valeur située entre 2,5 et 3,0 kV pour garantir une formation d'ozone minimale dans le plasma.

19. Procédé pour désodoriser les chaussures comprenant les étapes consistant à :
- fournir au moins un ensemble de décharge à pression atmosphérique (2, 22) comprenant une électrode haute tension (3, 23), une contre-électrode (4, 24) et au moins un élément diélectrique (5, 25) disposé entre elles, et comprenant en outre un générateur CA haute tension (6) connecté à ladite électrode haute tension (3, 23), moyennant quoi il comprend l'étape consistant à :
- fournir un boîtier (7) doté d'un orifice d'admission. (8) et d'un orifice d'échappement (9) de manière à définir un passage de fluide (10) s'étendant de l'orifice d'admission (8) vers l'orifice d'échappement (9), dans lequel ledit ensemble de décharge à pression atmosphérique (2, 22) est agencé dans le passage de fluide (10), ledit boîtier (7) étant formé et dimensionné de manière à pouvoir être complètement ou au moins partiellement inséré dans une chaussure :
- placer le boîtier dans une chaussure,
- alimenter le générateur CA haute tension (6),
- forcer un courant d'air le long du passage de fluide (10), et diffuser des vapeurs ou un gaz de substances prédéterminées dans ledit courant d'air de telle manière que le courant d'air est adapté pour transporter lesdites vapeurs ou ledit gaz vers l'ensemble de décharge à pression atmosphérique (2, 22) pour générer, à partir desdites vapeurs ou dudit gaz, des particules chimiquement hautement réactives, permettant ainsi l'écoulement desdites particules chimiquement hautement réactives à transporter directement dans la chaussure par l'intermédiaire de l'orifice d'échappement (9) pour décomposer des composés organiques présents dans la chaussure.
